# EUROPEAN PATENT APPLICATION

(11) **EP 3 839 039 A1**
(43) Date of publication of application: **23.06.2021**
(21) Application number: 19216569.4
(22) Date of filing: 16.12.2019
(51) Int. Cl.: C12N 1/04, A61K 35/66

(54) **PROVIDING BACTERIAL BIOMASS WITH IMPROVED STORAGE STABILITY**

(71) Applicant: 4D Pharma Research Limited, Aberdeen, Aberdeenshire AB25 2ZS (GB)
(72) Inventor: The designation of the inventor has not yet been filed
(74) Representative: Brand Murray Fuller LLP

(57) **Abstract**

Disclosed herein are processes for providing a concentrated biomass with a high concentration of viable bacteria. The invention also provides compositions obtained by these processes.

## Description

### SUMMARY

Disclosed herein are processes for providing a concentrated biomass with a high concentration of viable bacteria. The invention also provides compositions obtained by these processes. In some embodiments, a process comprises providing a fermentation medium comprising a first population of bacteria of interest and a growth medium. In some embodiments, a process comprises fermenting a first population of the bacteria of interest under growth conditions sufficient to provide a biomass comprising a second population of the bacteria of interest, where the second population comprises a larger number of the bacteria of interest than the first population of the bacteria of interest. In some embodiments, a process comprises concentrating a biomass. In some embodiments, a pH of a growth medium during a log phase of the fermenting is less than about 5.5. In some embodiments, a biomass comprising the second population of bacteria is washed with a mineral wash. In some embodiments, the process produces a viable concentrated biomass comprising the second population of the bacteria, as determined by centrifuging the biomass at about 10000G and measuring an optical density at 600 nm of a supernatant of the concentrated biomass, wherein the optical density is less than 0.1. In some embodiments, a pH adjustment step is conducted prior to or following the step of fermenting the first population of the bacteria of interest. In some embodiments, a fermentation step is conducted at a temperature of about 25°C to about 50°C. In some embodiments, a fermentation medium is agitated during the fermenting. In some embodiments, a degree of agitation is altered during the fermenting. In some embodiments, a degree of agitation is higher during the log phase of the fermenting than during the lag phase of the fermenting. In some embodiments, a pH of the fermentation medium is not controlled during the fermentation step. In some embodiments, a process can further comprise the step of harvesting the biomass following the fermentation step, where the harvested biomass comprises the second population of the bacteria of interest and a supernatant. In some embodiments, a biomass comprising the second population of the bacteria of interest is washed with a mineral wash. In some embodiments, a biomass is contacted with the mineral wash. In some embodiments, a mineral wash comprises metal ions in an amount of about 5wt% or lower, about 1wt% or lower, or about 0.5wt% or lower. In some embodiments, a mineral wash comprises metal ions in an amount of about 0.01wt% or higher, about 0.02wt% or higher, or about 0.05wt% or higher. In some embodiments, a mineral wash comprises metal ions from Group I and / or Group II of the periodic table. In some embodiments, a mineral wash comprises sodium, potassium, magnesium, calcium, lithium and / or beryllium ions. In some embodiments, a mineral wash comprises an antioxidant and / or a pH adjusting agent. In some embodiments, a washing step is carried out during the harvesting of the biomass. In some embodiments, a washing is performed at a volume ratio of fluid : biomass of from about 1:1 to about 20:1. In some embodiments, a process can further comprise the step of lyophilising the biomass to provide a lyophilised biomass. In some embodiments, a biomass is blended with a lyobuffer comprising a cryoprotectant, an antioxidant, a bulking agent, a surfactant and / or a buffer prior to a lyophilising. In some embodiments, a lyophilised composition comprising biomass and lyobuffer is provided in which the weight ratio of lyobuffer: biomass is at least 1:1. In some embodiments, a lyophilised compositions is provided comprising at least two of a monosaccharide, a disaccharide or a polysaccharide wherein optionally the monosaccharide, disaccharide or polysaccharide where present are provided at levels equal to or greater than the amount by weight of biomass present. In some embodiments, a lyophilised composition comprising biomass and cysteine and / or sodium glutamate is provided. In some embodiments, a lyophilised biomass has a viable cell count of at least about 1 x 10⁶ CFU/g, at least about 1 x 10⁷ CFU/g, at least about 1 x 10⁸ CFU/g or at least about 1 x 10⁹ CFU/g. In some embodiments, the viable cell count of the lyophilised biomass decreases by less than 1 x 10³ CFU/g, less than 1 x 10² CFU/g or less than 1 x 10¹ CFU/g when stored for three months at 5°C (±3°C) in moisture impermeable packaging. In some embodiments, a process can further comprise the step of blending the lyophilised biomass with one or more excipients, optionally diluents, stabilisers, growth stimulators, fillers, lubricants and / or glidants. In some embodiments, a viable cell count of the lyophilized biomass comprising the second population of the bacteria of interest is no more than 1 log lower than a viable cell count of the biomass comprising the second population of the bacteria of interest after the harvesting. In some embodiments, bacteria of interest belong to a genus selected from the group consisting of *Enterococcus, Pediococcus, Roseburia, Bacteroides, Bifidobacterium, Parabacteroides, Eubacterium, Faecolibacterium, Bariatricus, Megasphaera, Flavonifractor, Anaerotruncus, Ruminococcus, Pseudoflavonifractor, Clostridium, Coprococcus, Acetivibrio, Dorea, Blautia,* and *Erysipelatoclostridium.* In some embodiments, a bacteria of interest is not of the genus *Lactobacillus* or *Bifidobacterium.*

Also disclosed herein is a biomass washing fluid comprising a liquid medium comprising about 0.09% potassium phosphate, about 0.009% magnesium sulphate, about 0.009% calcium chloride, about 0.09% sodium chloride, and about 0.4% sodium bicarbonate. In some embodiments, a washing fluid comprises an antioxidant and / or a pH adjusting agent.

Also disclosed herein is a method of washing a biomass comprising a population of bacteria of interest, the process comprising contacting a biomass washing fluid as described herein with the biomass and optionally agitating the resulting mixture.

### BRIEF DESCRIPTION OF THE DRAWINGS

The novel features of exemplary embodiments are set forth with particularity in the appended claims. A better understanding of the features and advantages will be obtained by reference to the following detailed description that sets forth illustrative embodiments, in which the principles of exemplary embodiments are utilized, and the accompanying drawings of which:

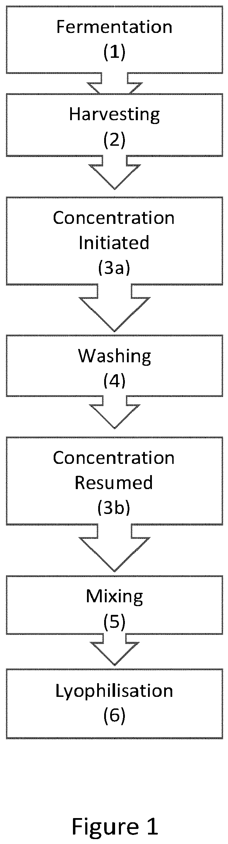

Fig. 1 depicts an exemplary workflow for producing a viable biomass of a population of a bacteria of interest. Such a biomass can be prepared by fermenting a population of bacteria without pH control, or by washing the biomass with a mineral wash.

### DETAILED DESCRIPTION

The present invention relates to pharmaceutical products comprising a live biotherapeutic product. The invention also relates to processes for manufacturing such pharmaceutical products and kits comprising such products.

The human intestine is thought to be sterile *in utero*, but it is exposed to a large variety of maternal and environmental microbes immediately after birth. Thereafter, a dynamic period of microbial colonization and succession occurs, which is influenced by factors such as delivery mode, environment, diet and host genotype, all of which impact upon the composition of the gut microbiota, particularly during early life. Subsequently, the microbiota stabilizes and becomes adult-like. The human gut microbiota contains more than 500-1000 different phylotypes belonging essentially to two major bacterial divisions, the Bacteroidetes and the Firmicutes. The successful symbiotic relationships arising from bacterial colonization of the human gut have yielded a wide variety of metabolic, structural, protective and other beneficial functions. The enhanced metabolic activities of the colonized gut ensure that otherwise indigestible dietary components are degraded with release of by-products providing an important nutrient source for the host. Similarly, the immunological importance of the gut microbiota is well-recognized and is exemplified in germfree animals which have an impaired immune system that is functionally reconstituted following the introduction of commensal bacteria.

Dramatic changes in microbiota composition have been documented in gastrointestinal disorders such as inflammatory bowel disease (IBD). For example, the levels of *Clostridium* cluster XIVa bacteria are reduced in IBD patients whilst numbers of *E. coli* are increased, suggesting a shift in the balance of symbionts and pathobionts within the gut. Interestingly, this microbial dysbiosis is also associated with imbalances in T effector cell populations.

Various strains have been proposed for use in the prevention, treatment and cure of various diseases. Examples of disclosures of the use of live bacterial organisms to treat physiological conditions include European Patent No. 1280541 (which discloses the use of hydrogenotrophic organisms in the treatment of a range of conditions including irritable bowel syndrome), European Patent No. 1448995 (which discloses the use of *Bacteroides thetaiotamicron* in the treatment of inflammatory diseases), European Patent Publication No. 2763685 (which discloses the use of *Roseburia hominis* as an immunoregulatory agent), European Patent No. 3209310 (which discloses the use of *Enterococcus gallinarum* as an anticancer therapy), and European Patent Publication No. 3206700 (which discloses the use of Bifidobacterium in the treatment of a range of autoimmune / inflammatory conditions, including severe asthma).

Some organisms are used as active principals in a class of pharmaceutical agents categorised by the US FDA as Live Biotherapeutic Products ("LBP"). In guidance published by the FDA in February 2012 and updated in 2016 (Guidance for Industry : Early Clinical Trials with Live Biotherapeutic Products: Chemistry, Manufacturing, and Control Information) LBP are defined as biological products that: 1) contain live organisms, such as bacteria; 2) are applicable to the prevention, treatment, or cure of a disease or condition of human beings; and 3) are not vaccines. It is further stated in the FDA's guidance document that (unlike probiotic products) LBP are subjected to the same rigorous scrutiny as pharmaceutical agents by regulatory bodies.

The present disclosure recognizes that, while LBP provide exciting and innovative therapies for a range of disease states, they remain challenging to formulate to pharmaceutical standards such that the active principles are viably delivered to the patient, especially after prolonged periods of storage prior to administration.

In conventional processes for producing non-pharmaceutical grade probiotics, samples from stored stocks of bacteria are grown up in fermenters to produce large volumes of biomass. This biomass is then harvested from the fermenter and typically concentrated to remove fermentation medium and other cellular products.

Depending on the intended end use of the bacteria of interest, the concentrated biomass may then be processed to render it processable and stable. In situations in which the bacteria are to be provided in solid dosage forms, the concentrated biomass may be lyophilised. Following lyophilisation, the lyophilised biomass may optionally be mixed with excipients. End products comprising the lyophilised biomass, for example, capsules or sachets, may then be produced.

The present disclosure recognizes that, while such processes have been used for preparing food supplements comprising probiotic organisms such as those belonging to the genera *Lactobacilii* or *Bifidobacterium*, optimisation is required for LBPs. The present disclosure also recognizes that conventional processes for preparing large-scale probiotic organisms are not generally applicable to live biotherapeutics for the following two reasons at least.

Firstly, the present disclosure recognizes that as LBPs are medicinal products, their potency must be predictable and carefully controlled; and excessive losses of viable cells during production and upon storage are not acceptable. As such stringent requirements are not applied to probiotics, conventional methods for probiotics manufacture are not typically appropriate for LBP production.

Secondly, the present disclosure recognizes that many organisms which are being developed for use as LBPs belong to different genera from those mentioned above and are phenotypically distinct from conventional probiotic bacteria. Thus, the formulation approaches with which an experienced formulator of probiotics will be familiar will need to be optimised to render them suitable for use with this new and highly regulated class of products.

Accordingly, the present disclosure recognizes a need in the art for improved processes for preparing products comprising live bacteria in which high levels of viable bacterial cells are maintained during the manufacture and storage of such products.

Thus, according to a first aspect of the present invention, there is provided a process for providing a concentrated biomass comprising: providing a fermentation medium comprising a first population of a bacteria of interest and a growth medium, conducting a fermentation step in which the first population of the bacteria of interest is fermented under growth conditions sufficient to provide a biomass comprising a second population of the bacteria of interest, the second population comprising a larger number of the bacteria of interest than the first population of the bacteria of interest, and concentrating the biomass, wherein pH of the fermentation medium is not controlled during fermentation step and / or wherein a biomass comprising the second population is washed.

The inventors have identified that the composition of the biomass harvested following fermentation can significantly influence the properties of downstream products prepared using that biomass. As demonstrated in the examples, the step of not controlling the pH of the fermenter leads to the production of a biomass which is more stable and processable. This finding is surprising and unexpected as it was previously believed that maintenance of the pH of the fermenter at what was thought to be an optimum pH for bacterial growth (e.g. at or near physiological pH) was a critical process condition. However, it has now been demonstrated, unexpectedly, that this is not the case.

The examples also demonstrate that washing the biomass permits the production of a more stable product. Without wishing to be bound by theory, this advantageous outcome is believed to be achieved as the washing step removes metabolites and / or cellular products in the biomass which would otherwise destabilise products prepared therefrom. Previously, in the preparation probiotic bacterial products, such steps were not thought to be essential and the addition of a further process step in an industrial-scale process was unattractive due to the additional time and effort that the additional washing step would add to the industrial process. However, the examples demonstrate that the advantages provided by the washing step outweigh any inconvenience arising from its performance.

In the first step of the process of the invention, a fermentation medium comprising a first population of a bacteria of interest and a growth medium are provided. In embodiments, this can be achieved by providing a growth chamber (e.g. a bioreactor or fermenter) with a bacterial stock and a growth medium. The bacterial stock can be pre-mixed with a portion of growth medium prior to being provided into the fermenter.

The fermentation medium comprises the bacteria of interest and the growth medium. In embodiments of the invention, the fermentation medium may further comprise additives, such as supplementary nutrition sources, proteins, amino acids, vitamins and / or antioxidants.

Any growth medium in which the bacteria of interest can be grown may be employed in the process of the present invention. Nutrient broths may be utilised as a growth medium. Those skilled in the art will be familiar with growth media for use in fermenting bacteria. Examples of such growth media include those comprising enzymatic digestion products of protein (referred to as peptone, tryptose and / or soytone growth media); yeast; one or more vitamins; amino acids; glycerol; and / or nutrition sources. Examples of growth media that can be employed in the process of the present invention include LB Medium, LB - Miller, LB - Lennox, LB - Low Salt, M9, Terrific Broth, EnPresso B, SOB Medium, SOC Medium, 2X YT Medium, NZCYM Broth, Yeast Nitrogen Base and / or NZ Amine® Broth, all commercialised by Sigma Aldrich.

The pH of the fermentation medium can be adjusted, prior to commencement of the fermentation step, for example to achieve a pH in the range of about 6 to about 8 or about 6.5 to about 7.5.

Any type of fermenter can be used in this step of the invention, provided that it is capable of housing a bacterial population and permitting and ideally promoting growth thereof. The skilled person will be familiar with such fermenters. In embodiments of the invention, the fermenter can be a reusable fermenter, for example one formed of and / or coated with stainless steel, ceramic, plastic or glass or can be a single use disposable fermenter, e.g. a vessel or bag formed of plastic such as polyethylene.

The fermenter can be a pilot scale fermenter (e.g. one having a capacity of from about 200ml, about 500ml or about 1L to about 5L, about 10L, about 15L or about 20L. In other embodiments, the fermenter can be a large-scale or commercial-scale fermenter, e.g. one having a capacity of about 100L, about 250L or about 500L to about 1000L, about 2000L, about 3000L, about 5000L or about 10000L.

The second step of the process of the invention is fermentation of the bacteria of interest under growth conditions sufficient to provide a biomass comprising a second population of the bacteria of interest.

As one skilled in the art would expect, fermentation of the bacteria of interest results in an increase in the number of that bacteria. The number of bacteria in the second population (CFU) can be at least about 100X greater, at least about 1000X greater, at least about 10000X greater, at least about 100000X greater, at least about 1 x 10⁵ X greater, at least about 1 x 10⁶ X greater or at least about 1 x 10⁷ X greater than in the first population (CFU).

The fermentation step can be conducted at a temperature of about 25°C to about 50°C, from about 30°C to about 45°C, or from about 35°C to about 40°C. The temperature can be constant throughout the fermentation step or can be altered.

In some cases, the fermentation medium is agitated during the fermentation step. This can be achieved using any apparatus or techniques known to those skilled in the art, for example through use of a rotary paddle, baffles formed within the fermenter, pumping the fermentation medium around the fermenter, or the like.

The skilled person will recognise that the fermentation of bacteria typically involves a lag phase (a period following the initiation of fermentation where no substantial increase of the bacterial population happens) and a log phase (a period following the lag phase in which there is an exponential increase in the bacterial population). He or she will also be familiar with techniques to determine when the lag phase ends, the log phase begins and the log phase ends, for example through the use of optical density, pH and / or viable cell count measurements.

In embodiments in which the fermentation medium is agitated, the degree of agitation can be altered during the fermentation step. For example, the degree of agitation can be higher during the log phase than during the lag phase. In arrangements in which agitation is achieved using a rotary paddle, the rotation speed during the log phase can be about 10rpm or more, about 20rpm, about 30rpm or more, about 40rpm or more or about 50rpm or more greater than the rotation speed during the lag phase.

While those skilled in the art will recognise that, conventionally, the pH of the fermentation medium should be carefully controlled during fermentation, in embodiments of the present invention, the pH of the fermentation medium is not controlled (i.e. the pH is allowed to naturally vary during the fermentation without the exogenous addition of a counter acid or base to maintain a particular pH). As demonstrated in the examples, not controlling the pH of the fermentation medium during fermentation unexpectedly provides advantageous results.

In some embodiments, the pH of the fermentation medium is not controlled for at least 10% of the lag phase. In some embodiments, the pH of the fermentation medium is not controlled for at least 20% of the lag phase. In some embodiments, the pH of the fermentation medium is not controlled for at least 30% of the lag phase. In some embodiments, the pH of the fermentation medium is not controlled for at least 40% of the lag phase. In some embodiments, the pH of the fermentation medium is not controlled for at least 50% of the lag phase. In some embodiments, the pH of the fermentation medium is not controlled for at least 60% of the lag phase. In some embodiments, the pH of the fermentation medium is not controlled for at least 70% of the lag phase. In some embodiments, the pH of the fermentation medium is not controlled for at least 80% of the lag phase. In some embodiments, the pH of the fermentation medium is not controlled for at least 90% of the lag phase. In some embodiments, the pH of the fermentation medium is not controlled for at least 95% of the lag phase. In some embodiments, the pH of the fermentation medium is not controlled for the entirety of the lag phase.

In some embodiments, the pH of the fermentation medium is not controlled for at least 10% of the log phase. In some embodiments, the pH of the fermentation medium is not controlled for at least 20% of the log phase. In some embodiments, the pH of the fermentation medium is not controlled for at least 30% of the log phase. In some embodiments, the pH of the fermentation medium is not controlled for at least 40% of the log phase. In some embodiments, the pH of the fermentation medium is not controlled for at least 50% of the log phase. In some embodiments, the pH of the fermentation medium is not controlled for at least 60% of the log phase. In some embodiments, the pH of the fermentation medium is not controlled for at least 70% of the log phase. In some embodiments, the pH of the fermentation medium is not controlled for at least 80% of the log phase. In some embodiments, the pH of the fermentation medium is not controlled for at least 90% of the log phase. In some embodiments, the pH of the fermentation medium is not controlled for at least 95% of the log phase. In some embodiments, the pH of the fermentation medium is not controlled for the entirety of the log phase.

In some embodiments, the pH of the fermentation medium is not controlled for at least 10% of the fermentation period. In some embodiments, the pH of the fermentation medium is not controlled for at least 20% of the fermentation period. In some embodiments, the pH of the fermentation medium is not controlled for at least 30% of the fermentation period. In some embodiments, the pH of the fermentation medium is not controlled for at least 40% of the fermentation period. In some embodiments, the pH of the fermentation medium is not controlled for at least 50% of the fermentation period. In some embodiments, the pH of the fermentation medium is not controlled for at least 60% of the fermentation period. In some embodiments, the pH of the fermentation medium is not controlled for at least 70% of the fermentation period. In some embodiments, the pH of the fermentation medium is not controlled for at least 80% of the fermentation period. In some embodiments, the pH of the fermentation medium is not controlled for at least 90% of the fermentation period. In some embodiments, the pH of the fermentation medium is not controlled for at least 95% of the fermentation period. In some embodiments, the pH of the fermentation medium is not controlled for the entirety of the fermentation period.

In some embodiments, the pH of the fermentation medium is not controlled until the pH of the fermentation medium reaches a threshold value, for example about pH 3, about pH 3.5, about pH 4, about pH 4.1, about pH 4.2, about pH 4.3, about pH 4.4, about pH 4.5, about pH 4.6, about pH 4.7, about pH 4.8, about pH 4.9 or about pH 5.

In embodiments of the invention, the pH of the fermentation medium is not controlled until the pH of the fermentation medium reaches a consistent level (to one decimal point) for a period of about 2 minutes, about 3 minutes, about 5 minutes, about 7 minutes, about 10 minutes, about 15 minutes, about 20 minutes, about 25 minutes or about 30 minutes.

The fermentation conditions are maintained until an desired yield of biomass is attained and / or until the end of the log phase. In embodiments of the invention, this can be for a period of up to about 0.5, about 1 or about 2 to about 12, about 18 or about 24 hours. The skilled addressee will be familiar with determining the point at which the log phase of bacterial growth in a fermenter commences and is concluded, for example through the use of optical density, pH and / or viable cell count measurements.

Irrespective of whether the pH of the fermentation medium is controlled during the fermentation step, a terminal pH adjustment step can be performed. In such embodiments, the terminal pH adjustment step can be performed at the end of the fermentation step, e.g. once a target yield of biomass is obtained and / or at the end of the log phase.

This can be achieved by adding a pH adjusting agent to alter (for example increase) the pH by about 0.5 or more, about 1.0 or more or about 1.5 or more. In embodiments of the invention, the pH of the mixture following the terminal pH adjustment step is about 5 to about 8, about 5.5 to about 7.5 or about 6 to about 7.

Where used, the pH adjusting agent can be basic. Suitable examples of pH adjusting agents which can be employed in the present invention include ammonia, sodium hydroxide, calcium hydroxide, calcium carbonate, and / or potassium hydroxide, magnesium hydroxide.

Upon completion of the fermentation step, the biomass can be harvested from the fermenter for further processing. Harvesting can be achieved by removing some or all of the biomass from the fermenter via an outlet provided therein, or via any other technique known to those skilled in the art. A pumping apparatus, e.g. a peristaltic pump (e.g. operated at a rate of at least about 0.5L per hour, at least about 1L per hour, at least about 2L per hour, at least about 5L per hour or at least about 10L per hour), can be employed to facilitate harvesting of the biomass from the fermenter. Additionally or alternatively, harvesting of the biomass can be achieved by feeding gas (e.g. an inert gas such as nitrogen) into the fermenter thus forcing out the biomass via an outlet.

The biomass harvested from the fermenter can be in liquid form. However, in alternative embodiments, the biomass can be in solid or semi-solid form.

In embodiments of the invention in which the biomass harvested from the fermenter is liquid, the biomass can comprise the bacteria of interest and a supernatant. The supernatant can comprise growth medium, metabolites produced by the bacteria of interest and / or cellular components of the bacteria of interest.

The process of the invention may comprise the step of concentrating biomass, i.e. to increase the concentration of the bacteria of interest therein. In some cases, the concentration step results in a 5X or greater, a 10X or greater, a 15X or greater or a 20X or greater concentration of the biomass.

The skilled person will be familiar with techniques which can be employed to concentrate biomass. In some embodiments of the invention, concentration of the biomass can be achieved via centrifugation, e.g. at a force of at least about 1000g, about 2000g or about 5000g and / or cross flow (tangential) filtration optionally using a microfiltration membrane (e.g. a membrane having pores of about 0.1 to about 1µm) and / or an ultrafiltration membrane (e.g. a membrane having pores of about 0.001µm to about 0.1µm).

The concentration step can be conducted at a temperature lower than the operating temperature of the fermenter during the fermentation step, but optionally above 0°C. In embodiments of the invention, the concentration step is conducted at a temperature of about 30°C or lower, about 20°C or lower or about 10°C or lower than the operating temperature of the fermenter during the fermentation step.

In embodiments of the invention, the biomass comprising the bacteria of interest can be subjected to a washing step. This can be performed prior to harvesting of the biomass from the fermenter (e.g. while the biomass is still contained within the fermenter or following harvesting of the biomass from the fermenter.

In embodiments of the invention in which the washing step is carried out following harvesting of the biomass from the fermenter, the washing step can be carried out prior to, during or following the step of concentrating the biomass.

For example, in embodiments in which the washing step is carried out during the step of concentrating the biomass, the concentration step can be initiated (e.g. centrifugation can be started) and then paused while the washing step is carried out, before the concentration step is resumed. Alternatively, the concentration step can be operated simultaneously with the washing step.

In the washing step of the invention, the biomass is contacted with a washing fluid and the resulting mixture optionally agitated. In embodiments, the washing fluid comprises a liquid medium, which can be an aqueous liquid medium. For example, the washing fluid can be water, saline solution (e.g. 0.9% saline solution) or a mineral solution.

In embodiments in which a mineral solution is used as the washing fluid. In some cases, the mineral solution can comprise metal ions. In such embodiments, the metal ions can comprise ions from Group I or Group II of the periodic table, such as sodium, potassium, magnesium, calcium, lithium and / or beryllium.

Where the washing fluid employed in the present invention comprises metal ions, these can be present at a concentration of about 0.01wt% or higher, about 0.02wt% or higher, or about 0.05wt% or higher. Additionally or alternatively, the concentration of metal ions present in the washing fluid can be about 5wt% or lower, about 2wt% or lower, about 1wt% or lower, about 0.5wt% or lower or about 0.2wt% or lower. In some embodiments, a metal ion can be present at a concentration of from about 0.01wt% to about 5wt%, from about 0.01wt% to about 4wt%, from about 0.01wt% to about 3wt%, from about 0.01wt% to about 2wt%, from about 0.01wt% to about 1wt%, from about 0.01wt% to about 0.5wt%, or from about 0.01wt% to about 0.1wt%.

The metal ions present in the washing fluid can be provided by adding metal salts into an aqueous medium. Any salt can be utilized to provide a source of a desired mineral in a wash solution. Such salts can include acetate, acrylate, adipate, alginate, aspartate, benzoate, benzenesulfonate, bisulfate, bisulfite, bitartrate, bromide, butyrate, butyn-1,4-dioate, camphorate, camphorsulfonate, caproate, caprylate, chlorobenzoate, chloride, citrate, cyclopentanepropionate, decanoate, digluconate, dihydrogenphosphate, dinitrobenzoate, dodecylsulfate, ethanesulfonate, formate, fumarate, glucoheptanoate, glycerophosphate, glycolate, hemisulfate, heptanoate, hexanoate, hexyne-1,6-dioate, hydroxybenzoate, γ-hydroxybutyrate, hydrochloride, hydrobromide, hydroiodide, 2-hydroxyethanesulfonate, iodide, isobutyrate, lactate, maleate, malonate, methanesulfonate, mandelate. metaphosphate, methanesulfonate, methoxybenzoate, methylbenzoate, monohydrogenphosphate, 1-napthalenesulfonate, 2-napthalenesulfonate, nicotinate, nitrate, palmoate, pectinate, persulfate, 3-phenylpropionate, phosphate, picrate, pivalate, propionate, pyrosulfate, pyrophosphate, propiolate, phthalate, phenylacetate, phenylbutyrate, propanesulfonate, salicylate, succinate, sulfate, sulfite, succinate, suberate, sebacate, sulfonate, tartrate, thiocyanate, tosylate, undeconate, and xylenesulfonate. In some embodiments, sodium ions can be provided by adding one or more of sodium chloride, sodium bicarbonate, sodium sulphate, sodium fluoride, sodium bromide, sodium carbonate or sodium amide to an aqueous medium. Potassium ions can be provided by adding one or more of potassium phosphate (e.g. potassium phosphate monobasic and / or potassium phosphate dibasic), potassium chloride, potassium hydroxide, potassium carbonate or potassium nitrate to an aqueous medium. Magnesium ions can be provided by adding one or more of magnesium chloride, magnesium carbonate, magnesium citrate, magnesium hydroxide, magnesium oxide or magnesium sulphate to an aqueous medium. Calcium ions can be provided by adding one or more of calcium chloride (e.g. monohydrate and / or dihydrate), calcium carbonate, calcium phosphate, calcium citrate or calcium lactate. Lithium ions can be provided by adding one or more of lithium chloride, lithium sulphate, lithium chloride, lithium bromide or lithium iodide to an aqueous medium. Beryllium ions can be provided by adding one or more of beryllium chloride, beryllium fluoride, beryllium hydroxide, beryllium sulphate, beryllium carbonate, beryllium nitrate or beryllium phosphate to an aqueous medium.

In embodiments of the invention, the washing solution can be a buffered solution. However, as explained above, it has been found that avoiding pH control of the biomass can provide advantages in terms of the stability of products prepared from the biomass, and thus in certain embodiments, the washing solution is not buffered.

The washing solution can additionally comprise other ingredients, for example an antioxidant (such as cysteine, arginine, ascorbic acid (and salts and esters thereof e.g. ascorbyl palmitate, sodium ascorbate), butylated agents such as butylated hydroxyanisole or butylated hydroxytoluene, citric acid, erythorbic acid, fumaric acid, glutamic acid, glutathione, malic acid, methionine, monothioglycerol, pentetic acid, metabisulfite (such as sodium metabisulfite, potassium metabisulfite), propionic acid, propyl gallate, uric acid, sodium formaldehyde sulfoxylate, sulphite (e.g. sodium sulphite), sodium thiosulfate, sulphur dioxide, thymol, tocopherol (free or esterified), uric acid (and salts thereof) and salts and / or esters thereof), pH adjusting agents (such as sulphate (e.g. ammonium sulphate) and / or one or more of the other pH adjusting agents discussed herein).

As is demonstrated in the examples below, the use of a washing agent having a composition as outlined herein advantageously and unexpectedly permits the production of pharmaceutical grade bacterial biomass while minimising the loss of viable cells normally observed in such processes. Thus, according to a further aspect of the present invention, there is provided a biomass washing solution comprising a liquid medium comprising metal ions at a concentration of about 5wt% or lower.

The present invention also provides a method of washing a biomass comprising a population of bacteria of interest, the process comprising contacting biomass washing fluid as discussed herein with the biomass and optionally agitating the resulting mixture.

In embodiments of the invention, the volume ratio of washing fluid : biomass can vary depending upon the composition of the washing fluid and the cellular concentration of the biomass. In embodiments, the volume of the washing fluid used in the washing step is equal to or greater than the volume of the biomass. Thus, the volume ratio of washing fluid : biomass can range from about 1:1 to about 2:1, about 3:1, about 4:1, about 5:1, about 10:1, about 20:1 or about 50:1.

Once the concentration step and washing step (if performed) have been completed, the concentrated biomass can then be lyophilised to produce a lyophilised composition comprising lyophilised biomass. Prior to lyophilisation, a lyobuffer composition comprising one or more excipients can be blended (collectively or separately) with the concentrated biomass. Such excipients can include:
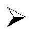 cryoprotectants (e.g. polyol such as ethylene glycol, sorbitol, propylene glycol, and / or glycerol; DMSO; skim milk; yeast extract; bovine serum albumin (BSA); starch hydrolysates; saccharides (including monosaccharides, disaccharides and / or polysaccharides) such as glucose, maltose, maltotriose, trehalose, mannitol, dextran, maltodextrin, lactose and / or sucrose; and / or amino acids such as cysteine, glutamic acid (optionally in the form of a salt, such as sodium glutamate), arginine and / or glycine),
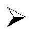 antioxidants (e.g cysteine, arginine, ascorbic acid (and salts and esters thereof e.g. ascorbyl palmitate, sodium ascorbate), butylated agents such as butylated hydroxyanisole or butylated hydroxytoluene, citric acid, erythorbic acid, fumaric acid, glutamic acid, glutathione, malic acid, methionine, monothioglycerol, pentetic acid, metabisulfite (such as sodium metabisulfite, potassium metabisulfite), propionic acid, propyl gallate, uric acid, sodium formaldehyde sulfoxylate, sulphite (e.g. sodium sulphite), sodium thiosulfate, sulphur dioxide, thymol, tocopherol (free or esterified), uric acid (and salts thereof) and salts and / or esters thereof),
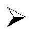 bulking agents (e.g. mannitol, maltodextrin and / or glycine),
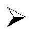 buffers (e.g. phosphate, citrate, tris and / or Hepes), and / or
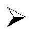 surfactants (e.g. polysorbate (such as those commercialised under the trade mark Tween) and / or sorbitan (such as those commercialised under the trade mark Span).

It has unexpectedly been found (as demonstrated in the examples) that the use of an excess of lyobuffer to biomass has the striking effect of i) maintaining high levels of viability of the biomass during the lyophilisation process, and also ii) and also upon prolonged storage. Thus, according to a further aspect of the present invention, there is provided a lyophilised composition comprising a lyophilised biomass comprising a bacteria of interest and a lyobuffer composition, wherein the dry mass of lyobuffer composition comprised in the lyophilised composition is equal to or greater than the dry mass of lyophilised biomass.

In certain embodiments, the dry weight ratio of lyobuffer composition : lyophilised biomass comprised in the lyophilised composition is about 1.5 : 1 or higher, about 2:1 or higher, about 3:1 or higher or about 4:1 or higher.

While conventional thinking in probiotic manufacture was that the lowest possible amount of lyobuffer should be employed in lyophilised compositions, so as to result in the highest potency probiotic composition, i.e. one having the highest number of probiotic cells per gram of composition, it has now been demonstrated that the compositions and processes of the present invention permits the manufacture of bacterial lyophilisates having greater numbers of viable bacteria than comparable compositions obtained using conventional formulation approaches.

The lyophilised biomass comprised the bacteria of interest. As discussed herein, the bacteria of interest may be any bacterial strain or strains which are useful as LBPs. Examples of bacterial genera to which those strain/s may belong are discussed extensively herein. In certain embodiments, the bacteria of interest are not bacterial strains of the genera Lactobacillus and / or Bifidobacterium and / or are not lactic acid bacteria. Additionally or alternatively, the bacteria of interest comprise bacterial strains which belong to the Bacteroides genus. For example, the bacteria of interest may consist exclusively of bacterial strains of the Bacteroides genus.

The lyobuffer composition comprised in the lyophilised composition of the present invention may comprise any of the lyobuffer excipients discussed above or elsewhere herein. For example, the lyobuffer composition may comprise a cryoprotectant, an antioxidant, a bulking agent, a buffer and / or a surfactant.

In preferred embodiments, a cryoprotectant is comprised within the lyobuffer composition. This may comprise a monosaccharide, a disaccharide or a polysaccharide.

In certain embodiments, the lyobuffer composition may comprise a monosaccharide and a disaccharide, a monosaccharide and a polysaccharide, or a disaccharide and a polysaccharide. In such embodiments, one or both of those saccharides are present at the same dry weight amount, or higher, than the dry weight of the lyophilised biomass. For example, the lyobuffer composition may comprise a disaccharide (e.g. sucrose) and a polysaccharide (e.g. maltodextrin) and each of the disaccharide and the polysaccharide may be present at levels (calculated on a dry weight basis) equal to or greater than the lyophilised biomass.

Additionally or alternatively, the lyobuffer composition may comprise an antioxidant (e.g. cysteine) and / or an amino acid (e.g. arginine, sodium glutamate and / or cysteine).

The material to be lyophilised can be loaded into containers. In some embodiments, the concentrated biomass can be loaded into individual vials. Alternatively, the concentrated biomass can be loaded into bulk containers, such as lyophilisation trays (plastic or metal) or the containers disclosed in International Patent Publication No. WO2019/012512, the contents of which are incorporated herein by reference.

The lyophilisation step can be carried out in a conventional lyophilisation apparatus with which the skilled person will be familiar. Any technique or apparatus known to those skilled in the art of lyophilisation can be employed. For example, the lyophilisation step can be conducted in lyophilisation apparatus such as pilot scale lyophilisation apparatus (e.g. freeze drying apparatus having an operating shelf area of about 0.1m² or higher, about 0.2m² or higher, about 0.5m² or about 2m² or lower, about 3m² or lower or about 4m² or lower) or commercial scale lyophilisation apparatus (e.g. freeze drying apparatus having an operating shelf area of about 5m² or higher, about 10m² or higher, or about 20m² or higher, or about 50m² or lower, about 100m² or lower, about 150m² or lower, or about 200m² or lower.

Lyophilised compositions of the present invention and / or obtained from the lyophilisation step of the present invention can comprise the bacteria of interest in an amount of at least about 1 x 10⁶ CFU/g, at least about 1 x 10⁷ CFU/g, at least about 1 x 10⁸ CFU/g, at least about 1 x 10⁹ CFU/g or at least about 1 x 10¹⁰ CFU/g.

Additionally or alternatively, the loss of viable bacteria (CFU/g) of the lyophilised compositions of the present invention and / or lyophilised compositions obtained from the process of the present invention, when stored at 5°C (±3°C) for 3 months in moisture impermeable packaging is equal to or less than 1 x 10³ CFU/g, equal to or less than 1 x 10² CFU/g or most preferably, equal to or less than about 1 x 10¹ CFU/g.

Once lyophilisation is complete, the lyophilised composition can be blended with one or more excipients before being provided in dosage forms. Such excipients can comprise diluents, stabilisers, growth stimulators, fillers, lubricants, glidants and the like. Examples of such suitable excipients can be found in the Handbook of Pharmaceutical Excipients. Acceptable excipients for therapeutic use are well known in the pharmaceutical art.

In some aspects, the pharmaceutical dosage form disclosed herein comprises one or more pharmaceutically acceptable excipients. Exemplary pharmaceutically acceptable excipients for the purposes of pharmaceutical compositions disclosed herein include, but are not limited to, binders, disintegrants, superdisintegrants, lubricants, diluents, fillers, flavors, glidants, sorbents, solubilizers, chelating agents, emulsifiers, thickening agents, dispersants, stabilizers, suspending agents, adsorbents, granulating agents, preservatives, buffers, coloring agents and sweeteners or combinations thereof. Examples of binders include microcrystalline cellulose, hydroxypropyl methylcellulose, carboxyvinyl polymer, polyvinylpyrrolidone, polyvinylpolypyrrolidone, carboxymethylcellulose calcium, carboxymethylcellulose sodium, ceratonia, chitosan, cottonseed oil, dextrates, dextrin, ethylcellulose, gelatin, glucose, glyceryl behenate, galactomannan polysaccharide, hydroxyethyl cellulose, hydroxyethylmethyl cellulose, hydroxypropyl cellulose, hypromellose, inulin, lactose, magnesium aluminum silicate, maltodextrin, methylcellulose, poloxamer, polycarbophil, polydextrose, polyethylene glycol, polyethylene oxide, polymethacrylates, sodium alginate, sorbitol, starch, sucrose, sunflower oil, vegetable oil, tocofersolan, zein, or combinations thereof. Examples of disintegrants include hydroxypropyl methylcellulose (HPMC), low substituted hydroxypropyl cellulose (L-HPC), croscarmellose sodium, sodium starch glycolate, lactose, magnesium aluminum silicate, methylcellulose, polacrilin potassium, sodium alginate, starch, or combinations thereof. Examples of a lubricant include stearic acid, sodium stearyl fumarate, glyceryl behenate, calcium stearate, glycerin monostearate, glyceryl palmitostearate, magnesium lauryl sulfate, mineral oil, palmitic acid, myristic acid, poloxamer, polyethylene glycol, sodium benzoate, sodium chloride, sodium lauryl sulfate, talc, zinc stearate, potassium benzoate, magnesium stearate or combinations thereof. Examples of diluents include talc, ammonium alginate, calcium carbonate, calcium lactate, calcium phosphate, calcium silicate, calcium sulfate, cellulose, cellulose acetate, corn starch, dextrates, dextrin, dextrose, erythritol, ethylcellulose, fructose, fumaric acid, glyceryl palmitostearate, isomalt, kaolin, lactitol, lactose, magnesium carbonate, magnesium oxide, maltodextrin, maltose, mannitol, microcrystalline cellulose, polydextrose, polymethacrylates, simethicone, sodium alginate, sodium chloride, sorbitol, starch, sucrose, sulfobutylether β-cyclodextrin, tragacanth, trehalose, xylitol, or combinations thereof.

Various useful fillers or diluents include, but are not limited to calcium phosphate, dibasic anhydrous, calcium phosphate, dibasic dihydrate, calcium phosphate tribasic, calcium sulphate, cellulose powdered, silicified microcrystailine cellulose, cellulose acetate, compressible sugar, confectioner's sugar, dextrates, dextrin, dextrose, fructose, kaolin, lactitol, lactose, lactose monohydrate, magnesium carbonate, magnesium oxide, maltodextrin, maltose, mannitol, microcrystailine cellulose, polydextrose, simethicone, sodium alginate, sodium chloride, sorbitol, starch, pregelatinized starch, sucrose, trehalose and xylitol, or mixtures thereof.

Examples of suitable lubricants include, but are not limited to, magnesium stearate, calcium stearate, zinc stearate, stearic acid, talc, glyceryl behenate, polyethylene glycol, polyethylene oxide polymers, sodium lauryl sulfate, magnesium lauryl sulfate, sodium oleate, sodium stearyl fumarate, DL-leucine, colloidal silica, and others as known in the art. In some embodiments a lubricant is magnesium stearate.

Various useful glidants include, but are not limited to, tribasic calcium phosphate, calcium silicate, cellulose, powdered, colloidal silicon dioxide, magnesium silicate, magnesium trisilicate, starch and talc, or mixtures thereof.

Pharmaceutically acceptable surfactants include, but are limited to both non-ionic and ionic surfactants suitable for use in pharmaceutical dosage forms. Ionic surfactants can include one or more of anionic, cationic or zwitterionic surfactants. Various useful surfactants include, but are not limited to, sodium lauryl sulfate, monooleate, monolaurate, monopalmitate, monostearate or another ester of olyoxyethylene sorbitane, sodium dioctylsulfosuccinate (DOSS), lecithin, stearyic alcohol, cetostearylic alcohol, cholesterol, polyoxyethylene ricin oil, polyoxyethylene fatty acid glycerides, and poloxamer.

Excipients employed in the present invention can comprise a prebiotic. The term "prebiotic" means a non-digestible ingredient that beneficially affects the LBP by selectively stimulating the growth and/or activity of one or a limited number of bacteria. Examples of prebiotics include oligosaccharides, fructooligosaccharides and galactooligosaccharides.

In embodiments of the invention, the process comprises the step of preparing a dosage form comprising the lyophilised biomass. Dosage forms comprising the lyophilised biomass can be prepared by punching or pressing tablet cores comprising the lyophilised biomass and optionally coating (e.g. enteric coating) them to provide tablets, encapsulating the lyophilised biomass into capsule shells to provide capsules, or providing the lyophilised biomass in sachets and sealing the sachets.

Examples of bacteria of interest, i.e. those which are processed according to the process of the invention and / or comprised in the lyophilised composition of the invention may include those belonging to the genera:
- Enterococcus (e.g Enterococcus gallinarum, Enterococcus caselliflavus, Enterococcus faecalis, or Enterococcus faecium),
- Pediococcus (e.g. Pediococcus acidilacticii)
- Roseburia (e.g. Roseburia hominis, Roseburia intestinalis or Roseburia inulinivorans),
- Bacteroides (e.g. Bacteroides thetaiotaomicron, Bacteroides massiliensis, Bacteroides fragilis, Bacteroides ovatus, Bacteroides vulgatus, Bacteroides dorei, Bacteroides uniformis or Bacteroides copricola),
- Bifidobacterium (e.g. Bifidobacterium breve, Bifidobacterium adolescentis or Bifidobacterium longum),
- Parabacteroides (e.g. Parabacteroides distasonis, Parabacteroides goldsteinii, Parabacteroides merdae, or Parabacteroides johnsonii),
- Eubacterium (e.g. Eubacterium contortum, fissicatena, Eubacterium eligens, Eubacterium hadrum, Eubacterium hallii, or Eubacterium rectale),
- Faecalibacterium (e.g. Faecalibacterium prausnitzii),
- Bariatricus (e.g. Bariatricus massiliensis),
- Megasphaera (e.g. Megasphaera massiliensis),
- Flavonifractor (e.g. Flavonifractor plautii),
- Anaerotruncus (e.g. Anaerotruncus colihominis),
- Ruminococcus (e.g. Ruminococcus torques, Ruminococcus gnavus, or Ruminococcus bromii),
- Pseudoflavonifractor (e.g. Pseudoflavonifractor capillosus),
- Clostridium (e.g. Clostridium nexile, Clostridium hylemonae, Clostridium butyricum, Clostridium tertium, Clostridium disporicum, Clostridium bifermentans, Clostridium inocuum, Clostridium mayombei, Clostridium bolteae, Clostridium bartletti, Clostridium symbiosum or Clostridium orbiscindens),
- Coprococcus (e.g. Coprococcus comes, or Coprococcus cattus),
- Acetivibrio (e.g. Acetovibrio ethanolgignens),
- Dorea (e.g. Dorea longicatena)
- Blautia (e.g. Blautia hydrogenotrophica, Blautia stercoris, Blautia wexlerae or Blautia producta), and / or
- Erysipelatoclostridium (e.g. Erysipelatoclostridium ramosum).

In specific embodiments, the bacteria of interest belong to the Bacteroides genera.

In embodiments of the invention, the bacteria present in the biomass are not conventional probiotic bacteria, e.g. they do not belong to the genera Lactobacillus, Bifidobacterium and / or are not lactic acid bacteria.

In certain embodiments, compositions of the invention may comprise more than one bacterial strain (such as a consortium of different bacterial strains). In such embodiments, compositions of the invention may comprise (in increasing preference) at least 3, at least 4, at least 5, at least 6, at least 7, at least 8, at least 9, at least 10, or at least 11 different bacterial strains. In such embodiments, the consortium may comprise at least three different *Parabacteroides* strains, such as a strain of *Parabacteroides distasonis*, a strain of *Parabacteroides johnsonii*, and a strain of *Parabacteroides gordonii.* In such embodiments, compositions of the invention preferably comprise at least 11 different bacterial strains, wherein said at least 11 different bacterial strains comprise a strain of *Parabacteroides distasonis*, a strain of *Parabacteroides johnsonii*, and a strain of *Parabacteroides gordonii.*

The invention further provides the following non-limiting embodiments:
1. A process for providing a concentrated biomass comprising: providing a fermentation medium comprising a first population of a bacteria of interest and a growth medium, conducting a fermentation step in which the first population of the bacteria of interest is fermented under growth conditions sufficient to provide a biomass comprising a second population of the bacteria of interest, the second population comprising a larger number of the bacteria of interest than the first population of the bacteria of interest, and concentrating the biomass, wherein the pH of the fermentation medium is not controlled during the fermentation step and/or wherein a biomass comprising the second population of bacteria is washed.
2. The process of Embodiment 1 wherein a pH adjustment step is conducted prior to or following the fermentation step.
3. The process of Embodiment 1 or 2, wherein the fermentation step is conducted at a temperature of about 25°C to about 50°C.
4. The process of any one of Embodiments 1 to 3, wherein the fermentation medium is agitated during the fermentation step.
5. The process of Embodiment 4, wherein the degree of agitation is altered during the fermentation step.
6. The process of Embodiment 5, wherein the degree of agitation is higher during the log phase of the fermentation step than during the lag phase of the fermentation step.
7. The process of any one of Embodiments 1 to 6, wherein the pH of the fermentation medium is not controlled during the fermentation step.
8. The process of any one of Embodiments 1 to 7, further comprising the step of harvesting the biomass following the fermentation step, wherein the harvested biomass comprises the second population of the bacteria of interest and a supernatant.
9. The process of any one of Embodiments 1 to 8, wherein the biomass comprising the second population of the bacteria of interest is washed with a washing fluid.
10. The process of Embodiment 9, wherein the washing fluid is a mineral wash.
11. The process of Embodiment 9 or 10, wherein the washing fluid comprises metal ions in an amount of about 5wt% or lower, about 1wt% or lower, or about 0.5wt% or lower.
12. The process of any one of Embodiments 9 to 11, wherein the washing fluid comprises metal ions in an amount of about 0.01wt% or higher, about 0.02wt% or higher, or about 0.05wt% or higher.
13. The process of any one of Embodiments 9 to 12, wherein the washing fluid comprises metal ions from Group I and / or Group II of the periodic table.
14. The process of any one of Embodiments 9 to 13, wherein the washing fluid comprises sodium, potassium, magnesium, calcium, lithium and / or beryllium ions.
15. The process of any one of Embodiments 9 to 14, wherein the washing fluid comprises an antioxidant and / or a pH adjusting agent.
16. The process of any one of Embodiments 8 to 15, wherein the washing step is carried out during the concentration of the biomass.
17. The process of any one of Embodiments 8 to 15, wherein the washing step is carried out during the harvesting of the biomass.
18. The process of any one of Embodiments 1 to 17, wherein washing is performed at a volume ratio of washing fluid : biomass of from about 1:1 to about 20:1.
19. The process of any one of Embodiments 1 to 18, further comprising the step of lyophilising the biomass to provide a lyophilised composition comprising a lyophilised biomass.
20. The process of Embodiment 19, wherein, prior to the lyophilizing, the biomass is blended with a lyobuffer composition.
21. The process of Embodiment 20, wherein the lyobuffer composition comprises a cryoprotectant, an antioxidant, a bulking agent, a surfactant and / or a buffer.
22. The process of any one of Embodiments 19 to 21, wherein the lyophilised composition has a viable cell count of at least about 1 x 10⁶ CFU/g, at least about 1 x 10⁷ CFU/g, at least about 1 x 10⁸ CFU/g or at least about 1 x 10⁹ CFU/g.
23. The process of any one of Embodiments 19 to 22, further comprising the step of blending the lyophilised composition with one or more excipients, optionally diluents, stabilisers, growth stimulators, fillers, lubricants and / or glidants.
24. The process of any one of Embodiments **Error! Reference source not found.** to 23, wherein a viable cell count of bacteria of interest in the lyophilised composition is no more than 1 log lower than a viable cell count of the bacteria of interest in the biomass after the harvesting.
25. The process of any one of Embodiments 1 to 24, wherein the bacteria of interest belongs to the genus Enterococcus, Pediococcus, Roseburia, Bacteroides, Bifidobacterium, Parabacteroides, Eubacterium, Faecalibacterium, Bariatricus, Megasphaera, Flavonifractor, Anaerotruncus, Ruminococcus, Pseudoflavonifractor, Clostridium, Coprococcus, Acetivibrio, Dorea, Blautia, or Erysipelatoclostridium.
26. The process of any one of Embodiments 1 to 25, wherein the bacteria of interest belongs to the Bacteroides genus.
27. The process of any one of Embodiments 1 to 26, wherein the bacteria of interest does not belong to the genus Lactobacillus or Bifidobacterium.
28. A biomass washing fluid comprising a liquid medium comprising metal ions at a concentration of about 5wt% or lower, about 2wt% or lower or about 1wt% or lower.
29. The biomass washing fluid of Embodiment 28, comprising metal ions in an amount of about 0.01wt% or higher, about 0.02wt% or higher, or about 0.05wt% or higher.
30. The biomass washing fluid of Embodiment 28 or 29 wherein the washing fluid comprises metal ions from Group I and / or Group II of the periodic table.
31. The biomass washing fluid of any one of Embodiments 28 to 30, wherein the metal ions from Group I and / or Group II of the periodic table are selected from sodium, potassium, calcium and / or magnesium or salts thereof.
32. The biomass washing fluid of any one of Embodiments 28 to 31 comprising at least two ofpotassium phosphate, magnesium sulphate, calcium chloride, sodium chloride, or sodium bicarbonate.
33. The biomass washing fluid of any one of Embodiments 28 to 32, wherein the biomass washing fluid comprises an antioxidant and / or a pH adjusting agent.
34. A method of washing a biomass comprising a population of bacteria of interest, the process comprising contacting the biomass washing fluid of any one of Embodiments 28 to 33 with the biomass and optionally agitating the resulting mixture.
35. A lyophilised composition comprising a lyophilised biomass comprising a bacteria of interest and a lyobuffer composition, wherein the dry mass of lyobuffer composition comprised in the lyophilised composition is equal to or greater than the dry mass of lyophilised biomass.
36. The lyophilised composition of Embodiment 35, wherein the dry weight ratio of lyobuffer composition : lyophilised biomass comprised in the lyophilised composition is about 1.5 : 1 or higher, about 2:1 or higher, about 3:1 or higher or about 4:1 or higher.
37. The lyophilised composition of Embodiment 35 or 36, wherein the lyobuffer composition comprises a saccharide, wherein the dry mass of saccharide comprised in the lyophilised composition is equal to or greater than the dry mass of lyophilised biomass.
38. The lyophilised composition of Embodiment 37, wherein the lyobuffer composition comprises two saccharides, wherein the wherein the dry mass of each saccharide comprised in the lyophilised composition is equal to or greater than the dry mass of lyophilised biomass.
39. The lyophilised composition of Embodiment 38, wherein the two saccharides are a monosaccharide and a disaccharide, a monosaccharide and a polysaccharide or a disaccharide and a polysaccharide.
40. The lyophilised composition of Embodiment 38 or 39 wherein the two saccharides are sucrose and maltodextrin.
41. The lyophilised composition of any one of Embodiments 35 to 40, wherein the lyobuffer composition comprises sodium glutamate, cysteine and / or arginine.
42. The lyophilised composition of any one of Embodiments 35 to 41, comprising the bacteria of interest in an amount of at least about 1 x 10⁶ CFU/g, at least about 1 x 10⁷ CFU/g, at least about 1 x 10⁸ CFU/g, at least about 1 x 10⁹ CFU/g or at least about 1 x 10¹⁰ CFU/g.
43. The lyophilised composition of any one of Embodiments 35 to 42, wherein loss of viable bacteria (CFU/g) when stored at 5°C (±3°C) for 3 months in moisture impermeable packaging is equal to or less than 1 x 10³ CFU/g, equal to or less than 1 x 10² CFU/g or equal to or less than about 1 x 10¹ CFU/g.

### EXAMPLES

### Example 1- General Process

The process of the invention was carried out as depicted in Figure 1. More specifically, 900mL of a culture comprising a population of bacteria from the genus Bacteroides deposited under accession number NCIMB 42408 with the international depositary authority NCIMB, Ltd. (Ferguson Building, Aberdeen, AB21 9YA, Scotland) on 3rd December 2014 (described more fully in International Patent Publication No. WO2016/203217) was added to a pilot scale stainless steel fermenter. The fermenter had a capacity of 3 litres and was supplied with a yeast / soytone / BMSY based growth medium additionally comprising hemin and vitamin B12 prior to the addition of the bacterial culture and the pH of the resulting mixture was adjusted to 7.

Fermentation of the bacteria was then initiated (1) and the fermentation step lasted for 8 hours. The pH of the fermentation medium in the fermenter was not controlled. The fermenter was provided with a paddle to agitate the mixture. During the lag phase of the fermentation step, the paddle was rotated at 20rpm. Once the log phase of the fermentation step commenced (as determined by optical density and pH measurements rotation of the paddle was increased to 100rpm.

Upon completion of the fermentation step, once the log phase had completed (as determined by optical density (OD₆₀₀ₙₘ of 4to 5), pH (4.7) and viable cell count measurements), a terminal pH adjustment step was carried out in some experimental runs (as detailed below). This was achieved by adding ammonium solution at an amount to increase the pH to between 5 and 7.

The biomass (in liquid form) was then harvested (2) by pumping biomass out from the fermenter via an outlet at a rate of 2L per hour using a peristaltic pump. An alternative way to harvest biomass from the fermenter would be via the introduction of nitrogen gas into the fermenter, thus forcing out the biomass.

The harvested biomass was then subjected to a concentration step (3a) to reduce the volume of supernatant and increase the concentration of bacteria. This was achieved by centrifugation at 10000G, and at a temperature of 4°C for a period of 30 minutes. This reduced in a 20X concentration of the harvested biomass.

The concentration step was then paused and in some experimental runs (as detailed below) washing fluid added (4) at a volume ratio of washing fluid : harvested biomass of 5:1. The resulting mixture was stirred to mix the biomass and washing fluid. Concentration of the biomass was then resumed (3b) via centrifugation at 10000G for 30 minutes. The total concentration of the harvested biomass achieved during the concentration step (3a and 3b) was 20X.

The concentrated biomass was then mixed with cryoprotectant (5) at a weight ratio of biomass : cryoprotectant of around 2:1. 5mL aliquots of the resulting mixture were then added into glass vials and subjected to lyophilisation (6) at a temperature of -80°C. The viable cell count of the lyophilised biomass was then assessed.

### Example 2 - Effect of Washing Step

Four lyophilised bacterial compositions were prepared in accordance with Example 1, as summarised in the table below:

| | **Experimental Run** | | | |
|---|---|---|---|---|
| | **1** | **2** | **3** | **4** |
| Washing Step Performed? | No | No | Yes | Yes |
| Washing Fluid | N/A | | Mineral Content: | |
| | | | 0.09% Potassium Phosphate | |
| | | | 0.009% Magnesium Sulphate | |
| | | | 0.009% Calcium Chloride | |
| | | | 0.09% Sodium chloride | |
| | | | 0.4% Sodium Bicarbonate | |
| | | | Other Content: | |
| | | | 0.09% Ammonium Sulphate | |
| | | | 0.009% L-cysteine | |
| | | | hydrochloride | |
| Lyoprotectant Composiiton | 2% Sucrose, 1.4% Mannitol, 0.14% L-Cysteine, HCl, 0.05M HEPES | 2% Sucrose, 1.4% Mannitol, 0.05M HEPES | 2% Sucrose, 1.4% Mannitol, 0.14% L-Cysteine,HCl, 0.05M HEPES | 2% Sucrose, 1.4% Mannitol, 0.05M HEPES |
| Viable Cell Count of Biomass Following Centrifugation | 3.96E+09 | | 7.87E+09 | |
| pH of Biomass Following Centrifugation | 4.72 | | 6.27 | |
| Viable Cell Count of Biomass + Lyoprotectant Prior to Lyophilisation | 3.33E+08 | 2.51E+08 | 5.60E+09 | 5.47E+09 |
| pH of Biomass + Lyoprotectant Prior to Lyophilisation | 4.69 | 4.77 | 5.31 | 5.24 |
| Viable Cell Count of Biomass + Lyoprotectant After Lyophilisation | 1.37E+07 | 1.50E+07 | 2.61E+09 | 3.21E+09 |
| pH of Biomass + Lyoprotectant After Lyophilisation | 4.86 | 4.92 | 5.76 | 5.67 |

As can be seen from this example, in the experimental runs in which a washing step was carried out, the steps of i) preparing the mixture of biomass and lyoprotectant and ii) lyophilisation both resulted in substantially lower reductions in viable cell count as compared to those runs where no washing step was conducted. Further, a substantially higher (>2 log) viable cell count was obtained in the experimental runs in which a washing step was conducted.

### Example 3 - Effect of Washing Fluid Composition

Two lyophilised bacterial compositions were prepared in accordance with Example 1, as summarised in the table below:

| | **Experimental Run** | |
|---|---|---|
| | **1** | **2** |
| Washing Step Performed? | Yes | Yes |
| Washing Fluid | 0.9 NaCl | Mineral Content: |
| | | 0.09% Potassium Phosphate |
| | | 0.009% Magnesium Sulphate |
| | | 0.009% Calcium Chloride |
| | | 0.09% Sodium chloride |
| | | 0.4% Sodium Bicarbonate |
| | | Other Content: |
| | | 0.09% Ammonium Sulphate |
| | | 0.009% L-cysteine hydrochloride |
| Lyoprotectant Composition | 2% Sucrose, | |
| | 1.4% Mannitol, | |
| | 0.14% L-Cysteine, HCI, | |
| | 0.05M HEPES | |
| Viable Cell Count of Biomass Following Centrifugation | 4.90E+10 | 2.08E+10 |
| pH of Biomass Following Centrifugation | 4.9 | 6.3 |
| Viable Cell Count of Biomass + Lyoprotectant Prior to Lyophilisation | 1.27E+10 | 2.08E+10 |
| pH of Biomass + Lyoprotectant Prior to Lyophilisation | 4.83 | 4.9 |
| Viable Cell Count of Biomass + Lyoprotectant After Lyophilisation | 2.89E+08 | 6.06E+09 |
| pH of Biomass + Lyoprotectant After Lyophilisation | 5.26 | 4.74 |

In both of these experimental runs, acceptably high viable cell counts were observed in the final, lyophilised compositions. However, the degree of loss in experimental run 2 was slightly lower than that in experimental run 1, demonstrating the optimal performance of the mineral-based washing solution.

However, if the results observed for experimental run 1 are compared to those observed in experimental run 1 in Example 2 (both of those runs including identical lyoprotectant formulations; the only difference between them being the presence of absence of a washing step with 0.9% NaCl washing fluid), it can be seen that the use of a 0.9% NaCl washing fluid does result in a smaller reduction in viable cell count than if no washing step is performed.

### Example 4 - Effect of Washing Step

Six lyophilised bacterial compositions were prepared as summarised in the table below. The process for the preparation of experimental runs 4 to 6 was conducted in accordance with Example 1. The process via which experimental runs 1 to 3 was conducted was altered to include an additional pH adjustment step prior to lyophilisation; the purpose of this was to adjust the pH of the biomass / lyoprotectant mixture to be lyophilised to correspond to the pH of the corresponding mixture in experimental runs 4 to 6 thus rendering those runs comparable.

| | **Experimental Run** | | | | | |
|---|---|---|---|---|---|---|
| | **1** | **2** | **3** | **4** | **5** | **6** |
| Washing Step Performed? | No | No | No | Yes | Yes | Yes |
| Washing Fluid | N/A | | | Mineral Content: | | |
| | | | | 0.09% Potassium Phosphate | | |
| | | | | 0.009% Magnesium Sulphate | | |
| | | | | 0.009% Calcium Chloride | | |
| | | | | 0.09% Sodium chloride | | |
| | | | | 0.4% Sodium Bicarbonate | | |
| | | | | Other Content: | | |
| | | | | 0.09% Ammonium Sulphate | | |
| | | | | 0.009% L-cysteine hydrochloride | | |
| Lyoprotectant Composiiton | 4% Sucrose; 2% Yeast Extract; 0.2% L-cystein; 2% L-arginine; 2% Mannitol; 0.05M HEPES | 4% Trehalose; 0.2% L-cystein; 2% Glutamic acid monosodium salt; 2% Mannitol; 0.05M HEPES | 4% Sucrose; 0.2% L-cystein; 2% L-arginine; 2% Mannitol; 0.05M HEPES | 4% Sucrose; 2% Yeast Extract; 0.2% L-cystein; 2% L-arginine; 2% Mannitol; 0.05M HEPES | 4% Trehalose; 0.2% L-cystein; 2% Glutamic acid monosodium salt; 2% Mannitol; 0.05M HEPES | 4% Sucrose; 0.2% L-cystein; 2% L-arginine; 2% Mannitol; 0.05M HEPE |
| Viable Cell Count of Biomass Following Centrifugation | 2.20E+10 | | | 2.76E+10 | | |
| pH of Biomass Following Centrifugation | 4.89 | | | 6.70 | | |
| Viable Cell Count of Biomass + Lyoprotectant Prior to Lyophilisation | 2.19E+09 | 4.50E+08 | 4.10E+08 | 1.05E+10 | 1.45E+10 | 9.08E+09 |
| pH of Biomass + Lyoprotectant Prior to Lyophilisation | 5.21 | 5.35 | 5.25 | 6.85 | 6.94 | 6.34 |
| pH Adjustment Prior to Lyophilisation | 6.61 | 6.6 | 6.75 | N/A | N/A | N/A |
| Viable Cell Count of Biomass + Lyoprotectant After Lyophilisation | 9.84E+08 | 2.20E+08 | 8.74E+07 | 2.51E+09 | 3.34E+09 | 2.46E+09 |
| pH of Biomass + Lyoprotectant After Lyophilisation | 5.72 | 5.59 | 6.22 | 6.91 | 6.84 | 6.03 |

As is apparent from the table above, the inclusion of a washing step utilising a mineral-based washing solution consistently limits reductions in viable cell counts irrespective of the formulation of the lyoprotectant.

### Example 5 - Effect of pH Control During Fermentation

Two biomass loads were prepared via a fermentation step. For the first biomass load, the fermentation step was conducted in accordance with Example 1 above. In the second, the process was varied by introducing continuous pH control during the fermentation step, as with conventional bacterial fermentation processes. pH control was achieved by adding liquid ammonia when pH dropped below 7 to maintain the pH of the fermentation medium within the fermenter at a pH of around 7.

With both biomass loads, the biomass was harvested from the fermenter upon completion of the log phase. The biomass which was subjected to pH control during fermentation was highly viscous and difficult to effectively remove from the fermenter, while the other biomass load (not subjected to pH control during fermentation) was more easily harvested and processable.

Following harvesting, both biomass loads were centrifuged at 10000G to increase biomass concentration by 20%. Optical density was then assessed using a spectrophotometer. Optical density (OD₆₀₀ₙₘ) of the remaining solution (supernatant) of the biomass load fermented without pH control was 0.002 (indicative of a low loss of viable cells), whereas the optical density (OD₆₀₀ₙₘ) of the supernatant of biomass load fermented with pH control was 1.10 (demonstrating a significant loss of viable cells).

### Example 6 - Comparison of Bacteroides lyophilisation compositions

A biomass comprising a strain of *Bacteroides thetaiotamicron* (deposited under Accession number 42341 on 3 December 2014 at National Collections of Industrial, Food and Marine Bacteria (NCIMB)) was lyophilised using a lyobuffer composition i) according to the present invention, and ii) comprising conventionally used excipients. Prior to lyophilisation, the biomass was not subjected to a washing step as discussed in the preceding examples.

Following lyophilisation, the viability of the composition comprising the conventional lyobuffer was assessed and found to be no higher than 1 x 10⁹ CFU/g. This level is significantly below the target viability threshold for the lyophilisate of 1 x 10¹⁰ CFU/g. Accordingly, this conventional composition was not sufficient to enable a commercially viable product to be prepared.

In contrast, when a lyophilisate comprising a biomass of the above-mentioned strain of *Bacteroides thetaiotamicron* was prepared using an excess of lyobuffer composition of the invention, a lyophilisate having a viable cell count in excess of the target threshold figure was obtained. This was then stored at 5°C (±3°C) in moisture impermeable packaging (alu/alu sachets) and the viability of the obtained product was found to be 4 x 10¹⁰ CFU/g after one month's storage, and 3.2 x 10¹⁰ after three months' storage. Thus, the inventive formulation enabled a product having higher initial viability to be prepared, and also no unacceptable loss in viability was observed following several months' storage.

Concentration of components post lyophilisation (excluding residual moisture in lyophilizate):

| Component | Conventional Formulation | Inventive Formulation |
|---|---|---|
| Biomass | 82.7% | 18.3% |
| D (+) Sucrose | | 22.2% |
| L cysteine hydrochloride | | 1.1% |
| Sodium L glutamate | 2.8% | 13.9% |
| L arginine | | 16.7% |
| Maltodextrin DE9 | | 27.8% |
| Glucose monohydrate | 0.075% | |
| Select soytone | 0.075% | |
| Yeast extract | 0.075% | |
| Ammonium chloride | 0.23% | |
| Sodium chloride | 0.075% | |
| Potassium dihydrogen phosphate | 0.11% | |
| Sodium carbonate | 0.088% | |
| Trehalose dehydrate | 13.78% | |
| Dry Matter | 100% | 100% |

## Claims

1. A process for providing a concentrated biomass comprising: providing a fermentation medium comprising a first population of a bacteria of interest and a growth medium, conducting a fermentation step in which the first population of the bacteria of interest is fermented under growth conditions sufficient to provide a biomass comprising a second population of the bacteria of interest, the second population comprising a larger number of the bacteria of interest than the first population of the bacteria of interest, and concentrating the biomass, wherein the pH of the fermentation medium is not controlled during the fermentation step and/or wherein a biomass comprising the second population of bacteria is washed.

2. The process of Claim 1
a) wherein a pH adjustment step is conducted prior to or following the fermentation step; and/or
b) wherein the fermentation step is conducted at a temperature of about 25°C to about 50°C; and/or
c) wherein the fermentation medium is agitated during the fermentation step, optionally wherein the degree of agitation is altered during the fermentation step, further optionally wherein the degree of agitation is higher during the log phase of the fermentation step than during the lag phase of the fermentation step; and/or
d) wherein the pH of the fermentation medium is not controlled during the fermentation step; and/or
e) further comprising the step of harvesting the biomass following the fermentation step, wherein the harvested biomass comprises the second population of the bacteria of interest and a supernatant; and/or
f) wherein the biomass comprising the second population of the bacteria of interest is washed with a washing fluid; optionally wherein
i. the washing fluid is a mineral wash; and/or
ii. the washing fluid comprises metal ions in an amount of about 5wt% or lower, about 1wt% or lower, or about 0.5wt% or lower; and/or
iii. the washing fluid comprises metal ions in an amount of about 0.01wt% or higher, about 0.02wt% or higher, or about 0.05wt% or higher; and/or
iv. the washing fluid comprises metal ions from Group I and / or Group II of the periodic table; and/or
v. the washing fluid comprises sodium, potassium, magnesium, calcium, lithium and / or beryllium ions; and/or
vi. the washing fluid comprises an antioxidant and / or a pH adjusting agent; and/or
vii. the washing step is carried out during the concentration of the biomass or the washing step is carried out during the harvesting of the biomass;
and/or
g) wherein washing is performed at a volume ratio of washing fluid : biomass of from about 1:1 to about 20:1; and/or
h) further comprising the step of lyophilising the biomass to provide a lyophilised composition comprising a lyophilised biomass; optionally
i. wherein prior to the lyophilizing, the biomass is blended with a lyobuffer composition; for example wherein the lyobuffer composition comprises a cryoprotectant, an antioxidant, a bulking agent, a surfactant and / or a buffer; and/or
ii. wherein the lyophilised composition has a viable cell count of at least about 1 x 10⁶ CFU/g, at least about 1 x 10⁷ CFU/g, at least about 1 x 10⁸ CFU/g or at least about 1 x 10⁹ CFU/g; and/or
iii. further comprising the step of blending the lyophilised composition with one or more excipients, optionally diluents, stabilisers, growth stimulators, fillers, lubricants and / or glidants; and/or
iv. wherein a viable cell count of bacteria of interest in the lyophilised composition is no more than 1 log lower than a viable cell count of the bacteria of interest in the biomass after the harvesting;
and/or
i) wherein the bacteria of interest belongs to the genus *Enterococcus, Pediococcus, Roseburia, Bacteroides, Bifidobacterium, Parabacteroides, Eubacterium, Faecalibacterium, Bariatricus, Megasphaera, Flavonifractor, Anaerotruncus, Ruminococcus, Pseudoflavonifractor, Clostridium, Coprococcus, Acetivibrio, Dorea, Blautia,* or *Erysipelatoclostridium*; and/or
j) wherein the bacteria of interest belongs to the *Bacteroides* genus; and/or
k) wherein the bacteria of interest does not belong to the genus Lactobacillus or Bifidobacterium.

3. A biomass washing fluid comprising a liquid medium comprising metal ions at a concentration of about 5wt% or lower, about 2wt% or lower or about 1wt% or lower.

4. The biomass washing fluid of Claim 3,
a) comprising metal ions in an amount of about 0.01wt% or higher, about 0.02wt% or higher, or about 0.05wt% or higher and/or
b) wherein the washing fluid comprises metal ions from Group I and / or Group II of the periodic table; and/or
c) wherein the metal ions from Group I and / or Group II of the periodic table are selected from sodium, potassium, calcium and / or magnesium or salts thereof; and/or
d) comprising at least two of potassium phosphate, magnesium sulphate, calcium chloride, sodium chloride, or sodium bicarbonate; and/or
e) wherein the biomass washing fluid comprises an antioxidant and / or a pH adjusting agent.

5. A method of washing a biomass comprising a population of bacteria of interest, the process comprising contacting the biomass washing fluid of Claim 4 with the biomass and optionally agitating the resulting mixture.

6. A lyophilised composition comprising a lyophilised biomass comprising a bacteria of interest and a lyobuffer composition, wherein the dry mass of lyobuffer composition comprised in the lyophilised composition is equal to or greater than the dry mass of lyophilised biomass.

7. The lyophilised composition of Claim 6, wherein the dry weight ratio of lyobuffer composition : lyophilised biomass comprised in the lyophilised composition is about 1.5 : 1 or higher, about 2:1 or higher, about 3:1 or higher or about 4:1 or higher.

8. The lyophilised composition of Claim 6 or 7, wherein the lyobuffer composition comprises a saccharide, wherein the dry mass of saccharide comprised in the lyophilised composition is equal to or greater than the dry mass of lyophilised biomass.

9. The lyophilised composition of Claim 8, wherein the lyobuffer composition comprises two saccharides, wherein the wherein the dry mass of each saccharide comprised in the lyophilised composition is equal to or greater than the dry mass of lyophilised biomass.

10. The lyophilised composition of Claim 9, wherein the two saccharides are a monosaccharide and a disaccharide, a monosaccharide and a polysaccharide or a disaccharide and a polysaccharide.

11. The lyophilised composition of Claim 9 or 10 wherein the two saccharides are sucrose and maltodextrin.

12. The lyophilised composition of any one of Claims 6 to 11, wherein the lyobuffer composition comprises sodium glutamate, cysteine and / or arginine.

13. The lyophilised composition of any one of Claims 6 to 12, comprising the bacteria of interest in an amount of at least about 1 x 10⁶ CFU/g, at least about 1 x 10⁷ CFU/g, at least about 1 x 10⁸ CFU/g, at least about 1 x 10⁹ CFU/g or at least about 1 x 10¹⁰ CFU/g.

14. The lyophilised composition of any one of Claims 6 to 13, wherein loss of viable bacteria (CFU/g) when stored at 5°C (±3°C) for 3 months in moisture impermeable packaging is equal to or less than 1 x 10³ CFU/g, equal to or less than 1 x 10² CFU/g or equal to or less than about 1 x 10¹ CFU/g.
